Europäisches Patentamt

European Patent Office

Office européen des brevets

(11) Numéro de publication: **0 110 926**
**B1**

(12) **FASCICULE DE BREVET EUROPEEN**

(45) Date de publication du fascicule du brevet: **18.05.88**

(51) Int. Cl.⁴: **A 61 F 13/18**

(21) Numéro de dépôt: **83901716.7**

(22) Date de dépôt: **01.06.83**

(86) Numéro de dépôt international:
**PCT/FR83/00105**

(87) Numéro de publication internationale:
**WO 83/04178 08.12.83 Gazette 83/28**

(54) **ARTICLE D'HYGIENE A JETER POUR INCONTINENTS MASCULINS.**

(30) Priorité: **02.06.82 FR 8209591**

(43) Date de publication de la demande:
**20.06.84 Bulletin 84/25**

(45) Mention de la délivrance du brevet:
**18.05.88 Bulletin 88/20**

(84) Etats contractants désignés:
**AT BE CH DE FR GB LI LU NL SE**

(56) Documents cités:
**FR-A-2 289 131**
**FR-A-2 433 936**
**US-A-2 896 626**
**US-A-3 868 287**
**US-A-3 968 798**

(73) Titulaire: **KAYSERSBERG SA**
**Route de Lapoutroie**
**F-68240 Kaysersberg (FR)**

(72) Inventeur: **MITRANI, Sem**
**76, rue des Bergeronnettes**
**F-91130 Ris Orangis (FR)**

(74) Mandataire: **David, Daniel**
**KAYSERSBERG 54, avenue Hoche**
**F-75008 Paris (FR)**

Courier Press, Leamington Spa, England.

## Description

La présente invention concerne un article absorbant à jeter destiné aux incontinents et plus particulièrement aux adultes masculins.

De nombreuses solutions ont été proposées pour résoudre le problème de l'incontinence adulte, notamment masculine.

Un de ces palliatifs est fourni par une poche réceptacle d'urine adaptable à l'homme incontient. Divers types sont décrits dans les brevets, notamment dans le brevet français 2.312.228.

Ce brevet concerne une poche s'adaptant à un mini-slip, spécialement conçu pour cet usage, par l'intermédiaire de fermetures de type bouton.

Cette poche sert de logement aux organes masculins et de réceptacle aux liquides. Elle comprend une enveloppe extérieure en plastique imperméable et éventuellement un revêtement intérieur en matière absorbante.

Mais un sous-vêtement spécial est nécessaire, la poche ne s'adaptant qu'à ce dernier.

D'autres produits destinés aux incontinents sont des articles d'hygiène absorbants.

Ce sont en fait des couches ou couche-culottes adaptées à la taille des adultes. De tels articles sont décrits dans un certain nombre de brevets, notamment US—A—4.018.226, FR—A—2.419.039, FR—A—2.443.831, ou sont commercialisés.

L'un de ces produits, de forme rectangulaire, présente une configuration en pli creux, les plis étant maintenus par deux points de colle dans la partie médiane de l'article, dans la zone de l'entrejambe. Les plis s'ouvrent dans la zone de la taille du porteur, la fixation s'effectuant par un ruban adhésif dans cette zone.

Les articles, vêtements et couches décrits dans l'art antérieur ainsi que ceux qui sont commercialisés présentent un ou plusieurs inconvénients.

Les poches sont assez volumineuses, désagréables à porter. Leur usage convient mieux en milieu hospitalier qu'en milieu ambulatoire.

Les changes sont de grandes dimensions, de la taille d'une culotte d'adulte et par là même, en usage ambulatoire, sont désagréables à porter du fait de leur encombrement. De plus, comme les "poches", elles provoquent une certaine gêne du point de vue esthétique, à leur porteur, en raison de leur épaisseur.

Le remplacement d'un change souillé nécessite en milieu hospitalier, diverses manipulations de la part de l'infirmier obligé de soulever le malade pour le changer.

L'usage en ambulatoire d'un tel change n'est pas non plus aisé, la fixation se faisant à la taille. Retirer un change usagé implique différentes manipulations obligeant le porteur à ôter, au moins partiellement, le vêtement et/ou le sous-vêtement recouvrant le change.

De plus, le port d'un produit mouillé, sur une grande surface n'est pas agréable, même peu de temps. Enfin, l'existence de fuites possibles, outre le risque de dégats matériels, provoque un état de malaise psychologique chez l'incontinent.

L'article d'hygiène selon l'invention permet de surmonter ces inconvénients en offrant les avantages de la pochet et de la couche-culotte pour adultes sans en présenter les défauts.

Plus particulièrement, l'article d'hygiène selon l'invention est confortable en raison de ses dimensions réduites et de sa bonne adaptation à l'anatomie masculine. Il est d'usage aisé, tant en milieu hospitalier qu'ambulatoire car il est facile à poser et à enlever en raison, notamment, de ses faibles dimensions et de la commodité de sa fixation. De plus, il offre une grande étanchéité en raison de son pouvoir absorbant associé à son effet de poche lors de sa mise en place, procurant à l'incontinent un sentiment de sécurité. De plus, son adaptation à une gamme étendue de sous-vêtements et de vêtements, même de sport, ne nécessitant donc pas le port d'un vêtement spécial est encore un avantage que présente l'article d'hygiène selon l'invention.

La présente invention a pour objet un article d'hygiène à jeter pour l'absorption des liquides corporels, pour incontinent masculin, du type comprenant un matelas de matière absorbante disposé entre une feuille imperméable aux liquides, destinée à être située vis-à-vis des vêtements de l'utilisateur et une feuille perméable aux liquides, les bords de l'une étant solidarisés aux bords adjacents de l'autre, ledit article étant de forme générale sensiblement rectangulaire, comportant des rabats longitudinaux tournés vers la face du matelas recouverte de la feuille perméable aux liquides et à laquelle ils sont rattachés par leurs extrémités transversales, caractérisé en ce que les extrémités desdits rabats sont fixées sur toute, leur largeur, par soudage ou collage, aux extrémités correspondantes de la partie de la feuille perméable sur laquelle lesdits rabats sont repliés, de manière à former une poche, les deux rabats ménageant, en position écartée, une ouverture permettant l'adaptation de la poche à l'anatomie masculine.

La feuille extérieure imperméable et la feuille intérieure perméable sont avantageusement fixées ensemble dans leurs parties latérales selon les lisières longitudinales, de telle sorte que le rebord extérieur est constitué d'une feuille extérieure imperméable délimitant une zone médiane revêtue de la feuille intérieure perméable.

Les deux rebords longitudinaux rabattus l'un vers l'autre peuvent être disposés de façon à être sensiblement adjacents et situés de part et d'autre de l'axe de symétrie longitudinal de la couche. En variante, il peut subsister un écart entre les rebords ménageant une ouverture longitudinale de la poche quand l'article se présente sous une forme plane avant d'être mis en service. Dans une autre forme de réalisation de l'invention, les bords longitudinaux des rabats se recouvrent au moins partiellement.

Parmi les moyens de fixation, on peut citer les bandes adhésives, les traits de colle, la thermofusion à chaud ou tout autre moyen. Le moyen de fixation peut consister également en un pan transversal à chacune des extrémités de l'article. Le pan est constitué par la feuille extérieure imperméable adhérant à la feuille intérieure perméable, tout en ayant lui-même la configuration de l'ensemble de l'article, à savoir les bords longitudinaux rabattus sur la face intérieure perméable. Le matelas absorbant ne s'étend pas dans le pan, mais constitue la surface adjacente à celui-ci. Mais cette solution est peu avantageuse sur le plan industriel.

L'article d'hygiène selon l'invention comporte également au moins un élément adhésif fixé à la face extérieure de la feuille imperméable recouvrant la face externe du matelas absorbant, et situé de préférence dans la zone avant de l'article d'hygiène, notamment sur sa ligne médiane.

Un tel élément adhésif pourra être une bande adhésive protégée avant usage par un papier siliconé.

Cette bande aura une longueur pouvant atteindre la moitié de celle de l'article d'hygiène, allant, par exemple, de son centre jusqu'à la ligne de pliure du pan.

Lors de l'utilisation de l'article d'hygiène, cette bande adhésive permet de fixer aisément la partie avant de l'article d'hygiène au sous-vêtement, ou même au vêtement, du porteur.

La configuration de rabats longitudinaux maintenus aux extrémités, avec fixation au sous-vêtement par la partie inférieure extrême de l'article d'hygiène, a un effet de poche. Lors de la mise en place, les rabats longitudinaux de l'article d'hygiène sont tendus et écartés. La face inférieure de l'article d'hygiène s'incurve, l'ensemble formant une poche s'adaptant parfaitement bien à l'anatomie masculine.

De plus, l'association d'une enveloppe extérieure imperméable avec bords externes des rabats longitudinaux également imperméables assure un grand degré d'étanchéité à un tel article.

En outre, les éléments absorbants internes de l'article retiennent l'urine, et, en cas de saturation, l'effet de poche évite les fuites de liquide en faisant réceptacle.

Dans une forme de réalisation de l'invention, les rabats longitudinaux sont simples, c'est-à-dire obtenus par simple repli des parties latérales de l'article d'hygiène, selon une ligne de pliage parallèle à la direction longitudinale de l'article.

Dans la forme préférée de réalisation de l'invention, l'article d'hygiène comporte des rabats longitudinaux "doubles", de section en forme de S, obtenus par un double repli de chaque partie latérale de l'article d'hygiène, donnant à l'ensemble de l'article d'hygiène une configuration en pli creux. Dans ce cas, les panneaux adjacents à la bande centrale sont fixés, à leurs extrémités latérales, aux extrémités latérales de ladite bande centrale. Les panneaux externes peuvent être libres ou fixés de la même manière aux panneaux adjacents.

Un article selon l'invention est fabriqué selon un procédé connu en soi dans le domaine des articles absorbants.

Les feuilles perméables et imperméables, dans leurs extrémités longitudinales, peuvent ne pas être collées dans une première étape, mais seulement après formation de la configuration en pli creux ou pli simple, par thermosoudage.

La fixation de la feuille extérieure imperméable sur la feuille intérieure perméable dans les parties latérales de l'article d'hygiène, selon les lisières longitudinales, s'effectue également par collage ou thermosoudage.

L'examen de l'état de la technique révèle des dispositifs avec rabats longitudinaux:

Le brevet FR—A—2289131 (COLGATE-PALMO-LIVE) porte sur un change pour bébé dont le matelas comporte un plissage susceptible d'être déployé latéralement. Cependant à la différence de l'invention, l'objet de ce brevet est de réaliser un change avec une ceinture se conformant à la taille du bébé par des moyens élastiques reliant au niveau des extrémités les différents plis entre eux. Ces moyens élastiques ne sont pas comparables aux moyens de liaison entre les extrémités des rabats et la partie centrale de l'article qui ne remplissent pas la même fonction.

Le brevet US—A—3968798 (HOKANSON) porte sur une couche pour incontinent comportant deux rabats longitudinaux. Cependant ces rabats, fixés au matelas par leur milieu, ont pour seule fonction de constituer une deuxième épaisseur de matelas pour limiter les fuites latérales de la couche. Par ailleurs, celle-ci recouvre les parties avant et arrière du bassin de l'utilisateur. Pour ces raisons, elle ne peut remplir la fonction de poche, par ouverture des rabats, recherchée par l'invention.

Le brevet FR—A—2433936 (COLGATE-PALMO-LIVE) décrit une compresse absorbante destinée à être placée dans la région périnéale, comportant un tampon de base recouvert par deux tampons supérieurs adjacents, de manière à former un puits en forme de T renversé. Bien qu'en utilisation les deux tampons supérieurs s'écartent légèrement pour faciliter le passage des liquides, ceux-ci ne s'ouvrent jamais complètement car ils sont conformés de façon que leur face supérieure reste en contact avec la peau de l'utilisatrice, le tampon de base devant obligatoirement rester éloigné pour remplir correctement sa fonction. Cet arrangement n'est donc pas transposable à l'incontinence masculine.

Le brevet US—A—3868287 (LEWYCKYJ) décrit une couche pour bébé comportant deux rabats dont l'extrémité avant seulement, est fixée à la partie centrale du matelas. En utilisation la partie arrière est déployée de manière à offrir une grande surface et la partie avant reste pliée de manière à offrir une double épaisseur pour absorber l'urine. Là encore la fonction réservée aux rabats est différente de celle réservée aux rabats de l'invention.

L'invention sera mieux comprise et les avantages de l'invention apparaîtront mieux à l'aide de la description ci-après de deux exemples de réalisation non limitatifs de l'objet de l'invention et des dessins annexés dans lesquels:

la figure 1 est une vue de dessus d'un article selon l'invention, avant pliage,

la figure 2 est une vue en coupe longitudinale selon AA de l'article de la figure 1,

la figure 3 est une vue en perspective d'un article selon l'invention, après pliage,

la figure 4 est une vue en coupe transversale selon BB de l'article de la figure 3,

la figure 5 est une vue de détail d'un article selon l'invention, après pliage et rabattement des extrémités.

la figure 6 est une vue en perspective d'un article selon la figure 5, préparé en vue de son utilisation.

La figure 7 est une vue en perspective d'un article selon la figure 3, préparé en vue de sont utilisation.

la figure 8 est une vue en coupe transversale d'une variante de l'invention,

la figure 9 est une vue en coupe transversale d'une autre variante de l'invention.

En se référant aux dessins sur les différentes figures, les références numériques désignent des éléments analogues.

La référence 1 désigne dans son ensemble l'article d'hygiène suivant l'invention.

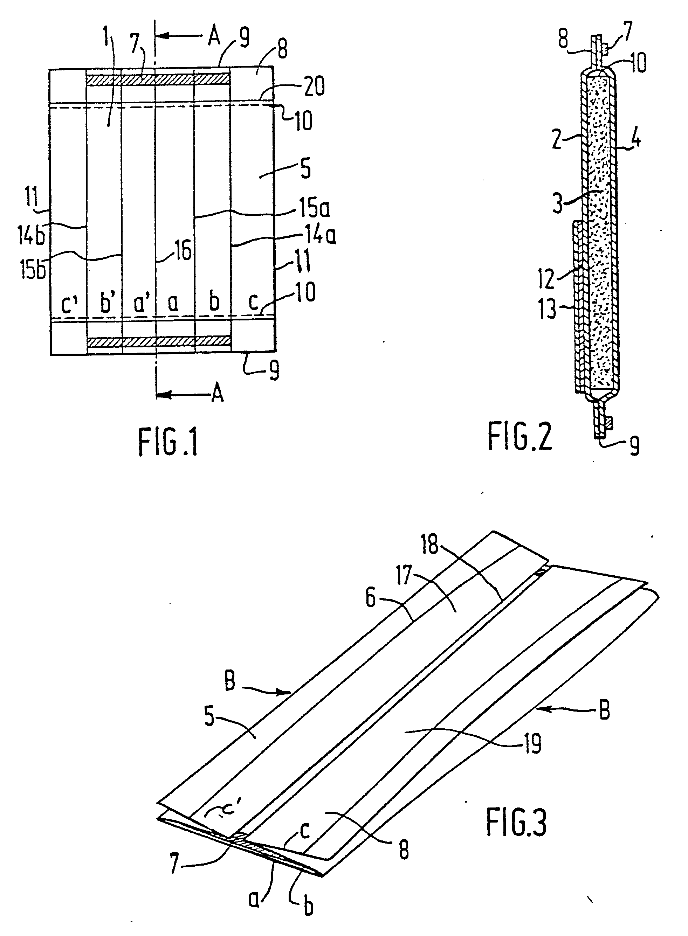

L'article 1 représenté sur les figures 1 et 2 est un article absorbant de configuration rectangulaire, comprenant une feuille rectangulaire extérieure 2, ou encore feuille support, constituée de matière plastique imperméable aux liquides, un matelas absorbant 3, également rectangulaire légèrement moins long que la feuille extérieure, mais centré sur elle, et une faulle intérieure 4 perméable aux liquides recouvrant le matelas absorbant.

La feuille 4 est également centrée sur la feuille extérieure 2 dont elle a de préférence la même longueur.

Le matelas absorbant 3 peut être, par exemple, un matelas de mousse de cellulose couramment dénommée "fluff".

La feuille imperméable 2 est en matière plastique telle que polyéthylène, polypropylène, poly-(chlorure de vinyle), etc.

La feuille perméable 4 est par exemple un non-tissé.

Sur le dessin de la figure 1, le matelas absorbant est limité à chaque extrémité par une ligne 10. La feuille intérieure 4 et la feuille imperméable 2 se rejoignent et sont soudées l'une à l'autre au-delà des bords 10 afin de former les pattes 8, délimitées elles-mêmes par les extrémités latérales 9 de l'article 1. Une ligne de pliage 20 peut être présente transversalement au niveau de ces pattes dans le cas où il est prévu de rabattre celles-ci.

Une bande adhésive 12 revêtue d'un papier protecteur siliconé 13 est apposée sur la feuille extérieure 2. Des lignes en traits fins 14a, 14b, 15a, 15b et 16 délimitent des panneaux de surface sensiblement égale a, b, c, et a', b', c', la ligne 16 selon l'axe AA partageant la surface en son milieu.

Un trait de colle 7 est appliqué transversalement dans la zone des pattes 8 et au moins dans la zone des panneaux a, a', b, b'.

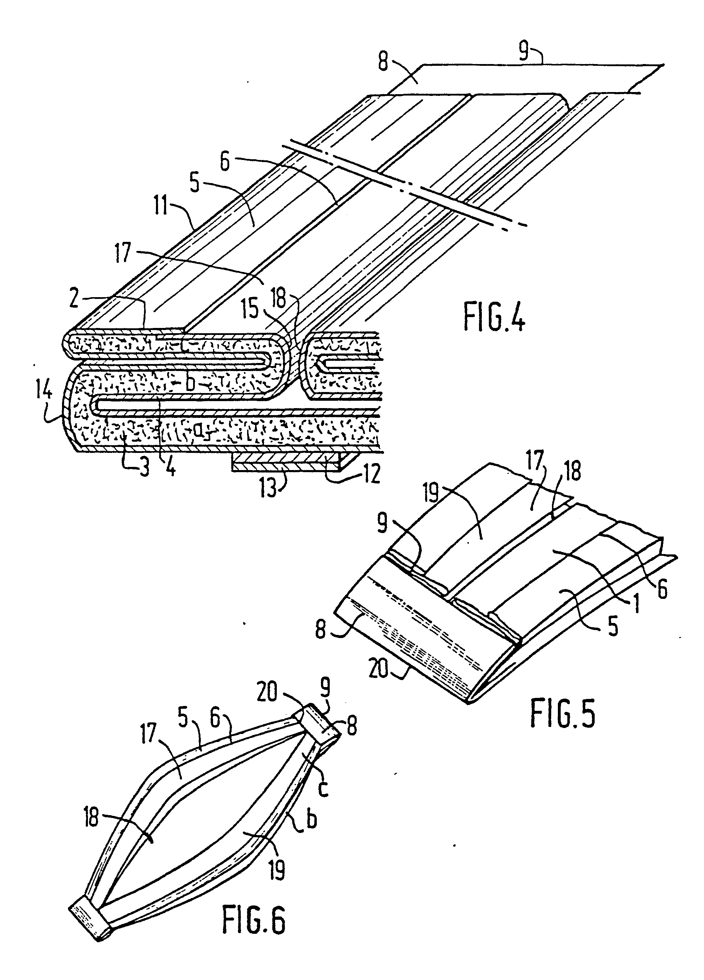

La figure 3 donne une vue d'ensemble de l'article 1 confectionné obtenu par pliage de l'article mis à plat décrit figure 1.

Le dessin de la figure 3 montre les panneaux c et c' constituant les faces supérieures 19 des rabats latéraux qui ont été pliés selon 14a, 14b et 15a, 15b, les panneaux c et c' recouvrant les panneaux b et b', eux mêmes recouvant les panneaux a et a', les faces supérieures des rabats c et c' arrivant bord à bord.

Le dessin de la figure 4 montre une vue en coupe de la figure 3 suivant BB, sur laquelle apparaissent les éléments décrits ci-avant, notamment les panneaux a, b, c, en leur position de pli double à section en S.

La ligne 6 figure le bord de la partie latérale 5 de la feuille extérieure imperméable 2 qui enveloppe le bord extérieur du matelas et vient par retour sur la face supérieure de celui-ci, revêtu de la feuille intérieure perméable. La partie latérale 5 est collée à la feuille intérieure perméable selon la bordure longitudinale 6. La partie centrale 17 de la face supérieure du panneau c est revêtue de la feuille intérieure perméable 4.

La bande adhésive 12 revêtue de son papier siliconé 13 est fixée à la feuille extérieure imperméable en la partie médiane de la face de l'article d'hygiène non tournée vers le corps.

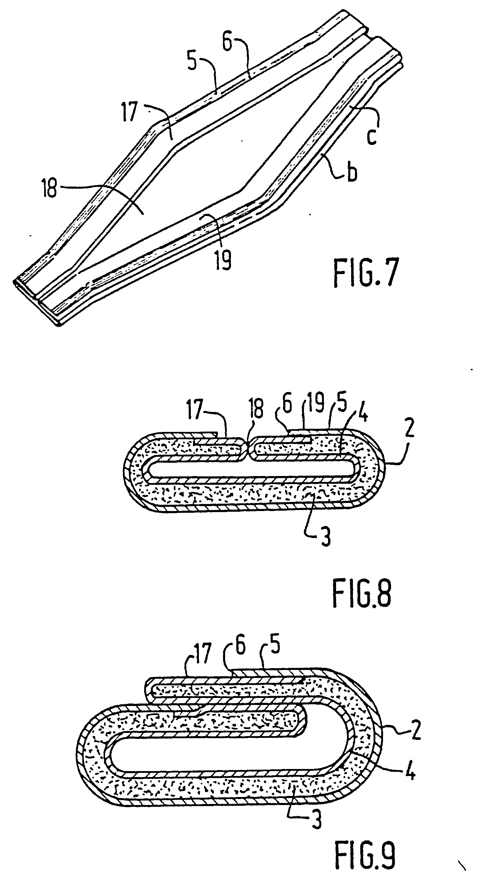

La figure 5 donne une vue de détail de l'article 1 lorsque la patte 8 est rabattue autour de la ligne de pliage 20.

La figure 6 est une vue en perspective d'un article selon l'invention préparé en vue de son utilisation. Les parties latérales 19 étant écartées laissent une ouverture 18 à l'article dont les panneaux a et b, déployés, forment poche.

La figure 7 est une vue en perspective d'un autre article selon l'invention décrite à la figure 3, les parties latérales 19 étant écartées laissant une ouverture 18.

Dans une variante de l'invention, les rabats longitudinaux sont simples, c'est-à-dire à une face, sans repli sur eux mêmes. La figure 8 donne une coupe de l'article selon cette variante. Le panneau b recouvre le panneau a. Il n'y a pas de panneau c. La feuille perméable 4 est rabattue au delà du bord du matelas absorbant 3 en constituant la partie centrale 17 perméable, fixée à la feuille extérieure imperméable 2 selon la lisière 6 de la partie latérale 19.

Dans une autre variante de l'invention, les rabats longitudinaux, qu'ils soient simples ou doubles, se recouvrent au moins partiellement. La figure 9 représente la section d'un article selon cette variante, les rabats étant simples. Ils sont superposés dans la partie centrale mais cette disposition peut présenter l'inconvénient, avec un matelas assez épais, d'avoir une épaisseur trop grande. Tel n'est pas le cas si les rabats sont simples et l'on appréciera, dans cette variante de l'invention, l'avantage que présente une telle configuration du point de vue d'une étanchéité accrue.

Il est préférable que les zones de l'article d'hygiène adjacentes à l'ouverture 18 de la poche restent perméables, alors que les parties extérieures 5 sont imperméables. C'est pourquoi, dans l'article d'hygiène, la feuille extérieure imperméable est prolongée au delà du bord 11 ou s'arrête en deça du bord, selon que le rabat longitudinal est constitué d'un pli double à section en S ou d'un pli simple.

Dans le cas d'un pli simple, qu'il y ait ou non superposition des deux bordures, c'est donc la feuille perméable qui est rabattue au dela du bord, comme représenté en coupe sur la figure 8.

Dans le cas d'un pli double à section en S, c'est la feuille imperméable qui est rabattue au delà du bord latéral de l'article d'hygiène, comme représenté en coupe dans la figure 4.

Un article selon l'invention ou l'une de ses variantes présente l'avantage d'avoir des dimensions réduites.

A titre indicatif, mais non limitatif, un article selon l'invention aura une longueur de 27 cm, une largeur de 9 cm, une épaisseur de matelas de 1 cm. Les rabats longitudinaux, simples ou doubles, auront, dans un tel article, une largeur de 4,5 cm, la bordure longitudinale imperméable extérieure une largeur de 2,5 cm.

## Revendications

1. Article d'hygiène à jeter pour l'absorption de liquides corporels, pour incontinent masculin, du type comprenant un matelas de matière absorbante (3) disposé entre une feuille imperméable aux liquides (2), destinée à être située vis-à-vis des vêtements de l'utilisateur et une feuille perméable aux liquides (4), les bords de l'une étant solidarisés aux bords adjacents de l'autre, ledit article étant de forme générale sensiblement rectangulaire, comportant latéralement des rabats longitudinaux (b, b') tournés vers la face du matelas recouverte de la feuille perméable aux liquides (4) et à laquelle ils sont rattachés par leurs extrémités transversales (8), caractérisé en ce que lesdites extrémités transversales (8) des rabats (b, b') sont fixées sur toute leur largeur, par soudage ou collage (7), aux extrémités correspondantes (9) de la partie (a, a') de la feuille perméable (4) sur laquelle lesdit rabats sont replies, de manière à former une poche sensiblement étanche, les deux rabats longitudinaux (b, b') menageant, en position écartée, une ouverture permettant l'adaptation de la poche à l'anatomie masculine.

2. Article d'hygiène selon la revendication 1, caractérisé en ce que les rabats longitudinaux (b, b') sont obtenus par pliage simple des parties latérales de l'article selon des lignes de pliage (15a, 15b) parallèles à la direction longitudinale de l'article, la feuille imperméable (2) recouvrant partiellement lesdits rabats (b, b').

3. Article d'hygiène selon l'une des revendications 1 ou 2, caractérisé en ce que les rabats longitudinaux sont des rabats doubles (c, c', b, b') à section en forme de S, la feuille imperméable (2) recouvrant partiellement les parties des rabats (c, c') venant en contact avec l'utilisateur.

4. Article d'hygiène selon l'une des revendications précédentes, caractérisé en ce que les bords internes (15) des rabats longitudinaux sont adjacents.

5. Article d'hygiène selon l'une des revendications 1 à 4, caractérisé en ce que les rabats longitudinaux (b, b') se recouvrent au moins partiellement.

6. Article d'hygiène selon l'une des revendications 1 à 4, caractérisé en ce que les bords internes (15) des rabats longitudinaux (b, b') laissent un intervalle entre eux.

7. Article d'hygiène selon l'une des revendications précédentes, caractérisé en ce qu'il comporte au moins un élément adhésif (12) situé sur la feuille extérieure imperméable (2).

## Patentansprüche

1. Hygienischer Wegwerfartikel zur Absorption von Körperflüssigkeit des inkontingenten Mannes, mit einem aus absorbierendem Material (3) bestehenden Polster, das zwischen einer für Flüssigkeit undurchlässigen Schicht (2), die der Kleidung des Trägers zugewandt ist, und einer flüssigkeitsdurchlässigen Schicht (4) angeordnet ist, wobei die Ränder der einen an den Rändern der anderen zusammengehalten sind, der Artikel allgemein die Form eines Rechtecks aufweist sowie seitliche längliche Falze (b, b'), die gegen die Fläche des Polsters gerichtet sind, die von der flüssigkeitsdurchlässigen Schicht (4) bedeckt ist und die an den Querenden angeheftet sind, dadurch gekennzeichnet, daß die Querenden (8) der Falze (b, b') über ihre ganze Erstreckung mittels Schweißen oder Kleben (7) an den entsprechenden Enden (9) des entsprechenden Abschnitts (a, a') auf der flüssigkeitsdurchlässigen Schicht (4) festgelegt sind, wobei die Falze in der Weise zurückgelegt sind, daß sie eine im wesentlichen dichte Tasche bilden, wobei die beiden länglichen Falze (b, b') beabstandet eine Öffnung bilden, die die Anpassung der Tasche an die männliche Anatomie erlaubt.

2. Hygienischer Artikel nach Anspruch 1, dadurch gekennzeichnet, daß die länglichen Falze (b, b') durch einfaches Falten seitlicher Abschnitte des Artikels entlang von Faltlinien (15a, 15b) parallel zur Längsrichtung des Artikels erhalten werden, wobei die undurchlässige Schicht die Falze (b, b') teilweise bedeckt.

3. Hygienischer Artikel nach Anspruch 1 oder 2, dadurch gekennzeichnet, daß die länglichen Falze (c, c', b, b') doppelt sind und im Schnitt ein S bilden, wobei die undurchlässige Schicht (2) teilweise die Teile (c, c') der Falze bedeckt, die in Berührung mit dem Träger kommen.

4. Hygienischer Artikel nach einem der vorangegangenen Ansprüche, durch gekennzeichnet, daß die inneren Ränder (15) der länglichen Falze benachbart sind.

5. Hygienischer Artikel nach einem der Ansprüche 1 bis 4, dadurch gekennzeichnet, daß die länglichen Falze (b, b') sich teilweise überdecken.

6. Hygienischer Artikel nach einem der Ansprüche 1 bis 4, dadurch gekennzeichnet, daß die inneren Ränder (15) der länglichen Artikel (b, b') zwischen sich einen Zwischenraum freilassen.

7. Hygienischer Artikel nach einem der vorangehenden Ansprüche, dadurch gekennzeichnet, daß er mindestens ein Klebeelement (12) auf der Außenseite der flüssigkeitsundurchlässigen Schicht (2) aufweist.

## Claims

1. Disposable hygiene article for the absorption of bodily liquids, for an incontient male, of the type comprising a padding of absorbent material (3) disposed between a sheet which is impermeable to liquids (2), intended to be positioned facing the user's clothing, and a sheet which is permeable to liquids (4), the edges of one being fixed to the adjacent edges of the other and the said article being of substantially rectangular flaps (b, b') turned towards the face of the padding covered by the sheet which is permeable to liquids (4) and to which they are attached by their transverse ends (8), characterised in that the said transverse ends (8) of the folds (b, b') are fixed over their entire width, by welding or adhesion (7), to the corresponding ends (9) of the part (a, a') of the permeable sheet (4) on which the said flaps are folded back, in a manner such as to form a substantially leaktight pouch, the two longitudinal flaps (b, b') providing, when moved apart, an aperture permitting the pouch to be adapted to the male anatomy.

2. Hygiene article according to Claim 1, characterised in that the longitudinal flaps (b, b') are obtained by folding the lateral parts of the article singly along fold lines (15a, 15b) parallel to the longitudinal direction of the article, the impermeable sheet (2) partially covering the said flaps (b, b').

3. Hygiene article according to one of Claims 1 and 2, characterised in that the longitudinal flaps are double flaps (c, c', b, b') of S-shaped section, the impermeable sheet (2) partially covering the parts of the flaps (c, c') which come in contact with the user.

4. Hygiene article according to one of the preceding claims, characterised in that the internal edges (15) of the longitudinal flaps are adjacent.

5. Hygiene article according to one of Claims 1 to 4, characterised in that the longitudinal flaps (b, b') overlap at least partially.

6. Hygiene article according to one of Claims 1 to 4, characterised in that the internal edges (15) of the longitudinal flaps (b, b') leave an interval between them.

7. Hygiene article according to one of the preceding claims, characterised in that it possesses at least one adhesive element (12) situated on the impermeable outer sheet (2).

FIG.1

FIG.2

FIG.3

FIG.4

FIG.5

FIG.6

FIG.7

FIG.8

FIG.9